(19) **Europäisches Patentamt / European Patent Office / Office européen des brevets**

(11) **EP 4 058 009 B1**

(12) # FASCICULE DE BREVET EUROPEEN

(45) Date de publication et mention de la délivrance du brevet:
**31.07.2024 Bulletin 2024/31**

(21) Numéro de dépôt: **20816110.9**

(22) Date de dépôt: **13.11.2020**

(51) Classification Internationale des Brevets (IPC):
*A61K 31/23* (2006.01)     *A61K 31/77* (2006.01)
*A61K 45/06* (2006.01)     *A61K 9/00* (2006.01)
*A61K 47/06* (2006.01)     *A61K 47/10* (2017.01)
*A61K 47/14* (2017.01)     *A61P 33/00* (2006.01)

(52) Classification Coopérative des Brevets (CPC):
(C-Sets disponibles)
**A61K 45/06; A61K 9/0014; A61K 31/23;
A61K 31/77; A61K 47/06; A61K 47/10;
A61K 47/14; A61P 33/00**          (Cont.)

(86) Numéro de dépôt international:
**PCT/EP2020/082100**

(87) Numéro de publication internationale:
**WO 2021/094561 (20.05.2021 Gazette 2021/20)**

(54) **SOLUTION HUILEUSE RINÇABLE POUR LUTTER CONTRE LES POUX**

SPÜLBARE ÖLIGE LÖSUNG ZUR BEKÄMPFUNG VON LÄUSEN

RINSABLE OILY SOLUTION FOR CONTROLLING LICE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **15.11.2019 FR 1912749**

(43) Date de publication de la demande:
**21.09.2022 Bulletin 2022/38**

(73) Titulaire: **DermoWave
9000064 Funchal (PT)**

(72) Inventeur: **PAGNOTTA, Nadine
33200 Bordeaux (FR)**

(74) Mandataire: **Aquinov
12, Cours Xavier Arnozan
33000 Bordeaux (FR)**

(56) Documents cités:
**EP-A2- 1 499 184       WO-A1-2019/053134
WO-A2-2004/037225    FR-A1- 2 949 182**

(52) Classification Coopérative des Brevets (CPC):
(Cont.)

C-Sets
**A61K 31/23, A61K 2300/00;**
**A61K 31/77, A61K 2300/00**

**Description**

La présente invention se rapporte au domaine de la lutte contre les poux et les lentes de tête.

Les poux sont des insectes hématophages pouvant être responsables de maladies parasitaires fréquentes et contagieuses appelées pédiculoses ou phitiriases. Trois types de poux en particulier sont responsables de ces ectoparasitoses :

- Pediculus humanus corporis, le pou de corps, vivant dans les coutures et les plis de vêtements, ne passant sur la peau que pour se nourrir, responsable de pédiculoses corporelles,
- Phtirius pubis, le pou du pubis et des autres régions du corps couvertes de poils grossiers ou morpion, responsable de phitiriases pubiennes, et
- Pediculus humanus capitis, le pou de tête, responsable de pédiculoses du cuir chevelu et/ou pédiculose de la tête.

La pédiculose de la tête est la plus répandue. C'est une affection endémique très fréquente touchant tous les pays et tous les milieux socio-économiques. Elle affecte surtout les enfants entre 5 et 11 ans.

Le pou de tête est un petit insecte de couleur grisâtre qui se nourrit du sang de son hôte trois à cinq fois par jour. Leurs oeufs ou lentes, de forme ovale, se fixent très solidement aux cheveux grâce à la spumaline matière collante secrétée par les lentes. Chaque lente est généralement collée à un cheveu, la plupart du temps tout près de la peau.

Les poux de tête ne peuvent ni sauter, ni voler et se transmettent surtout par un contact tête à tête. Ils peuvent être présents pendant plusieurs mois sur le cuir chevelu sans que le porteur ne s'en rende compte, ce qui nuit à la mise en place de mesures prophylactiques adéquates et favorise la transmission. L'infestation est habituellement sans symptôme durant les trois à quatre premières semaines, puis des démangeaisons apparaissent généralement, dues à la présence de substances allergènes dans la salive des parasites.

Dans le traitement des pédiculoses de la tête plusieurs approches peuvent être utilisées.

Il est notamment possible de peigner les cheveux pour éliminer les poux qui meurent rapidement une fois qu'ils sont séparés de leur hôte, mais cette solution ne permet d'éliminer qu'une partie des poux et ne peut aucunement être utilisée seule, sauf pour les enfants en très bas âge. Cette solution nécessite donc de réitérer l'opération de nombreuses fois.

L'approche la plus courante consiste à appliquer un insecticide sur le cuir chevelu. Les premiers traitements locaux qui ont été développés sont des produits à base d'insecticides, tels que des organophosphorés comme le malathion, des perméthrines ou des pyréthrines, associés à un agent synergique tel que le butoxyde de pipéronyle. Ces traitements, notamment ceux à base de malathion étaient considérés efficaces. Toutefois, il s'agit de produits très irritants pour le cuir chevelu, qui présentent en outre une odeur forte, désagréable et qui sont considérés comme toxiques et polluants pour l'environnement. En outre leur efficacité diminue avec le temps du fait d'une adaptation enzymatique, si bien que ce type de produit est délaissé.

Il existe également des traitements locaux à base de siloxanes notamment à base de diméticone, comme ceux décrit dans la demande WO-01/19190. Ces produits agissent en noyant les poux dans une phase huileuse, qui meurent par asphyxie. Néanmoins, ces produits ne sont pas suffisamment efficaces en particulier contre les lentes et nécessitent plusieurs applications répétées. En outre, ils rendent les cheveux gras, et sont très inflammables. Par ailleurs, les silicones utilisées ne sont pas biodégradables ce qui est dommageable pour l'environnement.

On connaît encore des produits insecticides à base de siloxanes et d'esters d'acides gras, tels que ceux décrits dans la demande EP-1.499.184. Ces produits agissent en dissolvant la chitine qui recouvre l'exosquelette du pou, provoquant sa déshydratation puis sa mort. Ils sont connus sous le nom commercial Resultz® constitués à 50% par de la Cyclométhicone, très inflammable, et à 50% par le Myristate d'isopropyle. Néanmoins, leur efficacité sur les poux adultes est très variable et leur pouvoir sur les lentes n'a pas été établi. En outre, ces produits ne peuvent pas être appliqués chez les enfants de moins de 4 ans et leur utilisation est fastidieuse puisqu'elle nécessite une application sur cheveux secs, un temps de pose d'au moins 8 heures et plusieurs shampoings des cheveux et du cuir chevelu pour être éliminé et diminuer le côté gras de la chevelure. Une nouvelle application, voire deux applications supplémentaires sont nécessaires pour éliminer les lentes écloses après un intervalle de 7 jours. Ces produits rendent également les cheveux gras et sont inflammables.

Il existe également des formulations de types microémulsion décrites dans la demande FR0955753, cependant ces formulations ne sont totalement efficaces sur les poux qu'après un temps d'une heure ce qui est long par rapport aux demandes actuelles des consommateurs et l'efficacité sur les lentes n'est pas complète.

Des produits à base de Chlorure de Sodium ont également été décrits. Si leur efficacité répond aux standards actuels ils laissent, du fait de leur contenu salin, un cheveu rêche et peu coiffable. Le caractère visqueux des formulations est incompatible avec une forme spray plus facile d'utilisation.

Plus récemment des formulations à base d'une solution huileuse constituée au moins par une phase dispersante huileuse dans laquelle est dispersée de l'alcool benzylique ont été décrite dans la demande WO2019/053134. Cependant, ces formulations notamment à base de particules de silice sont peu stables dans leur contenant. L'interaction des particules de silice avec le contenant et particulièrement les contenants en verre entraîne une gélification hétérogène de la formulation et de fait une mauvaise stabilité du produit. Par conséquent, la formulation est incompatible avec un produit qui soit facile d'utilisation pour le consommateur qui recherche un produit efficace et simple d'utilisation.

En outre, les particules de silice sont nanométriques (de taille inférieure au micron) ce qui pose un problème de toxicité de ces nano objets possiblement capables de passer la barrière cutanée et par conséquent, d'exercer une potentielle toxicité.

Enfin, la formulation décrite dans la présente demande est dépourvue d'alcool benzylique ce qui sur le plan de la tolérance et de la toxicité, est un avantage.

L'objectif de l'invention est de pallier les inconvénients de l'art antérieur, en proposant une solution facile d'utilisation, peu coûteuse, sans insecticide toxique, non inflammable et très efficace à la fois contre les poux de tête et les lentes.

Pour y répondre, l'invention propose une composition sous forme d'une solution huileuse rinçable pour son utilisation dans la prévention ou le traitement des pédiculoses, comprenant :

- au moins 70 % de Myristate d'isopropyle,
- au moins 7,5 % d'au moins un agent tensioactif ayant une balance hydrophile/lipophile inférieure ou égale à 5, et
- au moins 0,5 % de paraffine liquide,

les pourcentages étant donnés en poids du poids total de la composition, ladite composition ne comprenant ni particule de silice, ni alcool benzylique.

Avantageusement, la composition sous forme d'une solution huileuse selon l'invention présente une efficacité pédiculicide et lenticide totale et ce en une seule application d'une durée de moins de 15 minutes, préférentiellement d'une durée inférieure ou égale à 5 minutes. En outre, le produit est très facilement rinçable et d'une grande facilité d'utilisation. La faible viscosité des préparations permet de les proposer sous forme de lotions fluides ou de spray facile d'application.

L'invention a donc pour objet particulier la composition sous forme d'une solution huileuse rinçable pour une utilisation en application topique sur le cuir chevelu et les cheveux dans la prévention ou le traitement des pédiculoses de la tête chez l'homme ou sur le pelage animal dans la prévention ou le traitement des pédiculoses animales.

L'invention est définie dans les revendications ci-dessous.

## Définitions

Par « balance hydrophile/lipophile » ou « Hydrophilic-Lipophilic Balance » ou « équilibre hydrophile/lipophile » ou « HLB » au sens de l'invention on entend une grandeur caractéristique d'un tensio-actif qui est mesurée par une méthode, proposée en 1949 par Griffin permettant de chiffrer l'équilibre existant entre la partie hydrophile et la partie lipophile de la molécule de tensioactif; équilibre lié à la solubilité dans l'eau. L'échelle varie de 0 à 20 : plus la valeur est élevée, plus la solubilité dans l'eau est grande.

D'après la méthode de Davies, elle correspond à l'équation suivante [Math. 1]

$$HLB = \sum HLB_{groupes\ hydrophiles} - \sum HLB_{groupes\ hydrophobes} + 7$$

Les tensioactifs sont souvent utilisés en mélange et dans ce cas la valeur HLB d'un mélange binaire se calcule en première approximation par la relation linéaire suivante:

*HLB mélange = [(m1/m1+m2) x HLB1] +[(m2/m1+m2)xHLB2]*

suivant l'équation de Brochette (*Brochette, 1999: Elaboration et étude des émulsions. Techniques de l'ingénieur, traité Génie des procédés J2150 : 1-18* ) avec m1 : masse du tensioactif 1 dans la formulation, m2 : masse du tensioactif 2 dans la formulation, HLB1 : valeur HLB du tensioactif 1, HLB2 : valeur HLB du tensioactif 2

Les valeurs de HLB sont ainsi additives. Aussi par «balance globale hydrophile/lipophile » ou « balance hydrophile/lipophile globale » ou « équilibre hydrophile/lipophile global » ou « HLB global» au sens de l'invention on entend la somme des valeurs HLB des différents agents tensioactifs présents dans la solution.

Par « Myristate d'isopropyle » au sens de l'invention on entend le tétradécanoate de propan-2-yle (No CAS 110-27-0) qui est un ester de l'acide myristique et de l'isopropanol.

Par « rinçable » au sens de l'invention on entend l'action de rincer avec de l'eau du robinet la composition selon l'invention présente sur les cheveux ou la peau sans avoir à ajouter un savon ou un shampoing supplémentaire en plus de la composition.

Par « solution huileuse » au sens de l'invention on entend une formulation liquide huileuse obtenue sous agitation modérée à température ambiante des autre constituants de la composition tels que un agent tensioactif et de la paraffine liquide.

### Description détaillée de l'invention

L'invention a donc pour objet une composition sous forme d'une solution huileuse rinçable pour son utilisation dans la prévention ou le traitement des pédiculoses, comprenant :

- au moins 70 % de Myristate d'isopropyle,
- au moins 7,5 % d'au moins un agent tensioactif ayant une balance hydrophile/lipophile inférieure ou égale à 5, et
- au moins 0,5 % de paraffine liquide,

les pourcentages étant donnés en poids du poids total de la composition, ladite composition ne comprenant ni particule de silice, ni alcool benzylique.

Le Myristate d'isopropyle est une phase dispersante lipophile qui représente la base de la solution huileuse. Les autres constituants de la solution sont tous dissous sous agitation modérée à température ambiante ou sous une température modérée inférieure à 60°C dans cette phase. La solution huileuse ne comprend pas d'eau.

Préférentiellement, le Myristate d'isopropyle représente au moins 80% en poids du poids total de la composition selon l'invention, encore plus préférentiellement entre 83 et 92% en poids du poids total de la composition.

L'exosquelette des poux est constitué de chitine recouverte par une cire le protégeant. De par le mécanisme d'action du Myristate d'isopropyle qui dissout la cire recouvrant la chitine, elle ne remplit plus sa fonction de protection et provoque une déshydratation entraînant la mort des poux et de façon remarquable des lentes. Or, la couche de cire permet de retenir l'eau, et est indispensable à la survie du pou et des lentes. Ainsi, le Myristate d'isopropyle tue les poux par déshydration. Avantageusement, entre 83 et 92% de Myristate d'isopropyle en poids du poids total de la composition permet d'obtenir une très bonne efficacité sur les poux et lentes à cinq minutes après application de la composition selon l'invention.

La composition sous forme d'une solution huileuse rinçable comprend au moins 7,5% d'au moins un ou plusieurs agents tensioactifs ayant une balance hydrophile/lipophile inférieure ou égale à 5, de façon à rendre la solution facilement rinçable et par conséquent utilisable en une seule étape sans besoin d'application d'un shampoing additionel. De plus, l'agent tensioactif permet de diminuer la tension superficielle du milieu qui facilite la pénétration de la formulation au sein des poux et des lentes contribuant ainsi à renforcer l'efficacité de la formulation. L'agent tensioactif présent dans la solution selon l'invention est choisi parmi les agents tensioactifs cationiques, anioniques, amphotères et non ioniques ou leurs mélanges.

Ledit agent tensioactif présent dans la composition représente entre 7,5% et 15% en poids du poids total de ladite composition. Ceci est particulièrement avantageux pour conférer un caractère particulièrement rinçable à la formulation.

Avantageusement, l'agent tensioactif est le Propylene glycol monolaurate (Lauroglycol®) ayant une balance hydrophile/lipophile égale à 3.

La composition selon l'invention peut également comprendre une combinaison d'au moins deux agents tensioactifs dont la balance hydrophile/lipophyle de ladite combinaison est inférieure ou égale à 5. La combinaison d'au moins deux agents tensioactifs est particulièrement adaptée pour optimiser, améliorer la pénétration de la formulation au sein des poux et des lentes ainsi que de facilité le rinçage des cheveux. En effet, la nature des cheveux, notamment chez les humains, étant particulièrement hétérogène, la combinaison d'au moins deux agents tensioactifs vise à améliorer l'efficacité de rinçage de la solution après application.

Des exemples d'agents tensioactifs qui présentent une balance hydrophile/lipophile (HLB) inférieure ou égale à 5 et qui permettent d'obtenir une solution miscible avec le myristate d'isopropyle, sont donnés ci-après :

Glycol Distearate HLB = 1
Sorbitan Trioleate ou Span 85 HLB = 1.8
Propylene Glycol Isostearate HLB = 2.5
Glycol Stéarate HLB = 2.9
Lauroglycol™ 90, Propylene glycol monolaurate (type II) HLB 3
Sorbitan Sesquioleate HLB = 3.7
Glyceryl Stéarate HLB = 3.8
Lecithin HLB = 4

Sorbitan Oleate ou Span 80 HLB = 4.3
Sorbitan Monostearate NF HLB = 4.7
Sorbitan Stéarate HLB = 4.7
Sorbitan Isostearate HLB = 4.7
Steareth-2 HLB = 4.9
Oleth-2 HLB = 4.9
Lauroglycol™ FCC, Propylene glycol monolaurate (type I) HLB =3

Cette caractéristique, selon laquelle le ou les agents tensioactifs présents seul ou en association dans la composition présentent une balance hydrophile/lipophile (HLB) globale inférieure ou égale à 5, permet de conférer aux cheveux un aspect lisse brillant facilitant le décrochage des lentes contrairement aux tensio actifs de HLB plus élevés qui provoque un écartement des écailles rendant ainsi les cheveux électriques, rèches et peu coiffables.

La paraffine liquide permet un effet occlusif qui va emprisonner les poux et les asphyxier en obturant les orifices par lesquels ils respirent.

Préférentiellement, la paraffine liquide représente au moins 2% en poids du poids total de ladite composition. De manière particulièrement avantageuse, la paraffine liquide représente entre 2% et 4% en poids du poids total de la composition. A cette concentration la paraffine confère à la formulation un aspect agréable sur le plan cosmétique et contrecarre l'effet déséchant du aux fortes concentrations de myristate d'isopropyle

Selon une variante la composition selon l'invention est constituée exclusivement de :

- au moins 70 % de Myristate d'isopropyle
- au moins 7,5 % d'au moins un agent tensioactif ayant une balance hydrophile/lipophile inférieure ou égale à 5, et
- au moins 0,5 % de paraffine liquide,

les pourcentages étant donnés en poids du poids total de la composition.

Selon une autre variante, la composition selon l'invention peut également comprendre d'autres éléments solubilisés ou dispersés dans la solution huileuse, en plus du ou des agents tensioactifs. Il peut s'agir en particulier :

- d'un agent antioxydant, comme par exemple le butylhydroxytoluène, le butylhydroxyanisol ou le palmitate d'ascorbyle, qui permet de prévenir l'oxydation de la phase huileuse et ainsi son rancissement. Préférentiellement, l'agent antioxydant, lorsqu'il est présent dans la solution huileuse, représente moins de 1% en poids de la composition, et/ou
- d'une substance pédiculicide, qui permet d'améliorer l'efficacité de la composition, comme par exemple le malathion, les perméthrines, des essences végétales ou des huiles naturelles pédiculicides telles que l'essence d'anis, l'huile de l'arbre à thé, l'essence de myrte, l'essence de lavande, l'huile de neem et des inhibiteurs de la croissance des lentes tels que le Farnesol et le Nérolidol. Préfentiellement, la substance pédiculicide présente dans la composition, représente moins de 2% en poids de la composition et ce, afin d'éviter des effets indésirables, notamment toxicité, irritation.

- d'une substance répulsive contre les poux, comme par exemple l'essence de myrte, la citronnelle, l'éthyl butylacetylaminopropionate (IR 3535), le DET (Diéthyltoluamide), les huiles essentielles de citron, de lavande, de géranium rosat, de romarin cinéole, de céleri et de tea-tree. Préfentiellement, la substance répulsive présente dans la composition, représente moins de 5% en poids de la composition.
- d'un agent gélifiant, qui peut notamment être choisi parmi les copolymères de siloxane et de polyamides (comme par exemple Oléocraft LP20). Préférentiellement l'agent gélifiant lorsqu'il est présent dans la solution huileuse, représente moins de 30% en poids de la composition, encore plus préférentiellement entre 1 et 15%, et/ou
- d'un agent conservateur, comme par exemple l'acide benzoïque. Préférentiellement l'agent conservateur lorsqu'il est présent dans la solution huileuse, représente moins de 3% en poids de la composition, de façon préférée entre 0,1 et 3%.

La composition selon l'invention présente très préférentiellement une ou plusieurs de ces caractéristiques, de façon préférée toutes : stable, transparente voire translucide, lavable / rinçable à l'eau et très peu voire non irritante, non toxique, très peu voire non inflammable.

La composition selon l'invention peut être obtenue par tout procédé adapté. Il peut s'agir en particulier d'un procédé consistant en la mise en oeuvre des étapes successives suivantes à température ambiante :

- mélanger le ou les agents tensioactifs, la paraffine liquide et le Myristate d'isopropyle,
- éventuellement ajout d'un antioxydant dans la phase huileuse,
- éventuellement ajout d'un conservateur dans la phase huileuse.

Dans le cas où l'on souhaite gélifier la préparation, il est possible d'ajouter les étapes suivantes :

- Chauffer à environ 50°C et disperser le copolymère de polysiloxane et de polyamide,
- Faire refroidir la préparation.

La composition selon l'invention peut être utilisée en particulier sous forme de lotion ou sous forme de spray, ces deux formes étant particulièrement faciles d'utilisation pour des applications sur les cheveux et le cuir chevelu. Selon une variante, la composition, lorsqu'elle comprend un agent gélifiant, peut également se présenter sous forme de gel.

La composition selon l'invention, du fait de la paraffine liquide, permet d'agir très efficacement en noyant et en asphyxiant les poux. Elle présente également une rapidité d'action plus importante que les produits de l'art antérieur. En outre, la paraffine liquide permet de s'affranchir de l'effet desséchant de myristate d'isopropyle, de faciliter le coiffage et le décrochage des lentes.

Elle est utilisée comme produit de prévention et/ou de lutte contre les poux de tête et/ou contre les lentes, notamment pour être appliquée sur les cheveux et/ou le cuir chevelu ou sur le pelage d'animaux comme les chevaux.

La composition selon l'invention peut être appliquée indifféremment sur cheveux secs ou humides.

Un mode d'application particulier consiste à imprégner la chevelure sèche avec la composition selon l'invention et à rincer la chevelure avec de l'eau entre cinq minutes et une heure après l'application.

Avantageusement, il n'est pas nécessaire de répéter l'application après 7 jours étant donné la remarquable efficacité de la composition sur les lentes.

Selon un autre avantage, la composition selon l'invention laisse les cheveux soyeux, peignables et esthétiquement parfaits, contrairement aux produits de l'art antérieur qui laissent les cheveux gras, collants, rêches et qui nécessitent un shampoing pour éliminer le produit après application.

De plus, la composition selon l'invention présente un pouvoir de dissolution important notamment vis-à-vis de la spumaline, colle naturelle et forte sécrétée par les lentes qui rend difficile leur décrochage. Ainsi l'application des formulations permet une élimination facilitée des lentes.

L'invention est à présent illustrée par des exemples de compositions selon l'invention et par des essais d'efficacité pédiculicide et lenticide en particulier des essais comparatifs par rapport au produit commercialisé sous l'appelation Apaisyl Xpert®.

## Exemples de solutions huileuses selon l'invention

Exemple 1 : Formulation liquide

La formulation de la solution huileuse de l'exemple 1 est présentée dans le Tableau 1.

[Tableau 1]

| Formulation liquide | |
|---|---|
| Composé | |
| Myristate d'isopropyle | 90,5 |
| Lauroglycol® | 7,5 |
| Paraffine liquide | 2 |
| | 100 |
| Pourcentages exprimés W/W | |

Le procédé de fabrication est décrit ci après. Sous agitation modérée à température ambiante on mélange le Mysistate d'isopropyle au Lauroglycol® et à la paraffine liquide.

La formulation se présente alors sous la forme d'une lotion translucide facile d'application sur les cheveux et rinçable.

Exemple 2 : Formulation liquide

La formulation de la solution huileuse de l'exemple 2 est présentée dans le Tableau 2.

[Tableau 2]

| Formulation liquide | |
| --- | --- |
| Composé | |
| Myristate d'isopropyle | 83 |
| Lauroglycol® | 15 |
| Paraffine liquide | 2 |
| | 100 |
| Pourcentages exprimés W/W | |

Le procédé de fabrication est décrit ci-après. Sous agitation modérée à température ambiante on mélange le Myristate d'isopropyle, la Paraffine liquide et le Lauroglycol®.

La formulation se présente sous la forme d'une lotion translucide facile d'application sur les cheveux sous forme de lotion ou de spray et rinçable.

Exemple 3 : Formulation gélifiée

La formulation de la solution gélifiée de l'exemple 3 est présentée dans le Tableau 3.

[Tableau 3]

| Formulation gélifiée | |
| --- | --- |
| Composé | |
| Myristate d'isopropyle | 83,5 |
| Lauroglycol® | 7,5 |
| Paraffine liquide | 4 |
| SP Oleocraft LP-20 | 5 |
| | 100 |
| Pourcentages exprimés W/W | |

Le procédé de fabrication est décrit ci-après. Sous agitation énergique à une température de 40°C on mélange successivement le Myristate d'isopropyle au Lauroglycol®, la paraffine liquide et l'Oléocraft®.

La formulation se présente sous la forme d'une solution gélifiée.

Exemple 4 : Formulation liquide

La formulation de la solution huileuse de l'exemple 4 est présentée dans le Tableau 4.

[Tableau 4]

| Formulation liquide | |
| --- | --- |
| Composé | |
| Myristate d'isopropyle | 90,5 |
| Span 80® | 7,5 |
| Paraffine liquide | 2 |
| | 100 |
| Pourcentages exprimés W/W | |

Le procédé de fabrication est décrit ci-après. Sous agitation à température ambiante on mélange le Myristate d'isopropyle, le Span 80® et la paraffine liquide.

La formulation se présente sous la forme d'une solution translucide facile d'application en lotion ou en spray sur les cheveux et rinçable.

Exemple 5 : Formulation gélifiée

La formulation de la solution gélifiée de l'exemple 5 est présentée dans le Tableau 5.

[Tableau 5]

| Formulation gélifiée | |
|---|---|
| Composé | |
| Myristate d'isopropyle | 83,5 |
| Span 80® | 7,5 |
| Paraffine liquide | 4 |
| SP Oleocraft LP-20 | 5 |
| | 100 |
| Pourcentages exprimés W/W | |

Le procédé de fabrication est décrit ci-après. Sous agitation à température de 40°C on mélange successivement le Myristate d'isopropyle, la paraffine liquide, le Span80® à l'Oléocraft®.

La formulation se présente sous la forme d'une solution gélifiée.

Exemple 6 : Formulation liquide

La formulation de la solution huileuse de l'exemple 6 est présentée dans le Tableau 6.

[Tableau 6]

| Formulation liquide | |
|---|---|
| Composé | |
| Myristate d'isopropyle | 83 |
| Span 85® | 15 |
| Paraffine liquide | 2 |
| | 100 |
| Pourcentages exprimés W/W | |

Le procédé de fabrication est décrit ci-après. Sous agitation à température ambiante on mélange le Myristate d'isopropyle, le Span 85® et la paraffine liquide.

La formulation se présente sous la forme d'une solution translucide facile d'application en lotion ou en spray sur les cheveux et rinçable.

Ces formulations sont particulièrement adaptées à l'utilisation revendiquée.

**Essais d'efficacité de l'invention et comparaison avec une autre formulation**

Des essais d'efficacité pédiculicide et lenticide d'une composition selon l'exemple 1 en comparaison avec une formulation huileuse sous forme de microémulsion comprenant notamment de la paraffine liquide, du Myristate d'isopropyle et du PEG-4, commercialisée sous le nom Apaisyl® Anti-Poux Xpert et avec un contrôle ont été réalisés.

Les activités pédiculicides et ovicides de la composition Apaisyl Xpert® et de la composition selon l'exemple 1 ont été testées en laboratoire sur le pou du corps humain selon le mode opératoire suivant :

Les poux *(Pediculus humanus humanus)* sont élevés en laboratoire selon la méthode décrite par Cole (1966). Pour chaque essai, 50 poux (10 mâles, 10 femelles et 30 nymphes) ont été placés sur un disque de papier filtre blanc de 7 cm et exposés à 1 g de la préparation à l'essai. Les poux sont restés en contact avec la substance pendant 5 minutes. Ils ont été retirés du papier filtre, lavés à l'eau du robinet pendant 1 minute. Après le traitement, les poux ont été transférés sur un disque de papier filtre frais et incubés toute la nuit à une température et une humidité optimale. La mortalité a été déterminée après 24 heures.

Les poux ont été placés sur des cheveux humains tous les deux jours pendant 5 jours et laissés pendant 24-48 heures pour la ponte. Cinquante oeufs âgés de 2 à 6 jours ont été traités de la même façon que les poux. Le nombre d'oeufs éclos et non éclos a été compté après 10 jours.

Comme contrôle, on a utilisé des poux traités avec de l'alcool éthylique à 40 %. Chaque temps d'exposition a été testé en trois exemplaires.

Les résultats sont exprimés en pourcentage de poux morts.

Les compositions testées sont présentées dans le Tableau 7.

[Tableau 7]

| COMPOSE | Mortalité chez les poux (moyenne exprimée en %) après cinq minutes de contact et rinçage | Mortalité chez les lentes (moyenne exprimée en %) après cinq minutes de contact et rinçage |
|---|---|---|
| Apaisyl Xpert | 70.0 | 52 |
| Composition (Exemple 1) | 100 | 98 |
| Contrôle | 8.0 | 12.0 |

Pour la composition selon l'invention, on constate une parfaite efficacité pédiculicide et lenticide avec seulement 5 minutes de contact ce qui est remarquable et encore jamais inégalé pour un produit dépourvu d'insecticide.

Cette nouvelle composition selon l'invention permet une meilleure efficacité, 5 minutes au lieu d'une heure pour la plupart des produits concurrents, notamment Apaisyl Xpert®, une meilleure stabilité et une similarité sur le plan organoleptique.

## Revendications

1. Composition sous forme d'une solution huileuse rinçable pour son utilisation dans la prévention ou le traitement des pédiculoses, comprenant :

   - au moins 70 % de Myristate d'isopropyle,
   - au moins 7,5 % d'au moins un agent tensioactif ayant une balance hydrophile/lipophile inférieure ou égale à 5, et
   - au moins 0,5 % de paraffine liquide,

   les pourcentages étant donnés en poids du poids total de la composition,
   ladite composition ne comprenant ni particule de silice, ni d'alcool benzylique.

2. Composition pour son utilisation selon la revendication 1, **caractérisée en ce qu'**elle comprend au moins 80% de Myristate d'isopropyle, préférentiellement entre 83% et 92% de Myristate d'isopropyle.

3. Composition pour son utilisation selon l'une des revendications 1 ou 2, **caractérisée en ce que** l'agent tensioactif présent dans la composition représente entre 7,5% et 15% en poids de ladite composition.

4. Composition pour son utilisation selon l'une des revendications 1 à 3, **caractérisée en ce que** l'agent tensioactif est le propylene glycol monolaurate ayant une balance hydrophile/lipophyle égale à 5.

5. Composition pour son utilisation selon l'une des quelconques revendications précédentes, **caractérisée en ce que** la composition comprend une combinaison d'au moins deux agents tensioactifs dont la balance hydrophile/lipophile globale de ladite combinaison est inférieure ou égale à 5.

6. Composition pour son utilisation selon l'une des quelconques revendications précédentes, **caractérisée en ce que** la paraffine liquide représente au moins 2% en poids de ladite composition, préférentiellement entre 2% et 4% en poids de ladite composition.

7. Composition pour son utilisation selon l'une des quelconques revendications précédentes, **caractérisée en ce que** l'agent tensioactif ou la combinaison d'agents tensioactif est choisi parmi les agents tensioactifs cationiques, anioniques, amphotères et non ioniques.

8. Composition pour son utilisation selon l'une des quelconques revendications précédentes, **caractérisée en ce qu'**elle comprend également au moins un agent antioxydant, et/ou au moins une substance pédiculicide, et/ou au moins une substance répulsive contre les poux, et/ou un agent gélifiant, et/ou un agent conservateur.

9. Composition pour son utilisation selon la revendication 8, **caractérisée** en que l'agent antioxydant représente moins de 1% en poids de la composition et/ou est choisi parmi le butylhydroxytoluène, le butylhydroxyanisol et le palmitate d'ascorbyle.

10. Composition pour son utilisation selon la revendication 8, **caractérisée** en que la substance pédiculicide représente moins de 2% en poids de la composition et/ou est choisie parmi le malathion, les perméthrines, des essences végétales ou des huiles naturelles pédiculicides telles que l'essence d'anis, l'huile de l'arbre à thé, l'essence de myrte, l'essence de lavande, l'huile de neem et des inhibiteurs de la croissance des lentes tel que le Farnesol et le Nérolidol.

11. Composition pour son utilisation selon la revendication 8, **caractérisée** en que la substance répulsive représente moins de 5% en poids de la composition et/ou est choisie parmi l'essence de myrte, la citronnelle, l'éthyl butylacetylaminopropionate (IR 3535), le DET (Diéthyltoluamide), les huiles essentielles de citron, de lavande, de géranium rosat, de romarin cinéole, de célerie et de tea-tree.

12. Composition pour son utilisation selon l'une des quelconques revendications précédentes, **caractérisée en ce qu'**elle est utilisée sous forme de lotion ou de spray.

13. Composition pour son utilisation selon la revendication 8, **caractérisée en ce que** l'agent gélifiant représente 5% en poids de la composition et/ou est choisi parmi les copolymères de siloxane et de polyamides.

14. Composition pour son utilisation selon la revendication 8, **caractérisé en ce que** l'agent conservateur représente moins de 3% en poids de la composition.

15. Composition pour son utilisation selon l'une des quelconques revendications précédentes, **caractérisée en ce qu'**elle est utilisée pour une application topique sur le cuir chevelu et les cheveux dans la prévention ou le traitement des pédiculoses de la tête chez l'homme ou sur le pelage animal dans la prévention ou le traitement des pédiculoses animales.

**Patentansprüche**

1. Zusammensetzung in Form einer spülbaren öligen Lösung zur Verwendung bei der Vorbeugung oder der Behandlung von Pedikulose, umfassend:

   - zu mindestens 70 % Isopropylmyristat,
   - zu mindestens 7,5 % mindestens ein Tensid, das ein hydrophiles/lipophiles Gleichgewicht von weniger als oder gleich 5 besitzt, und
   - zu mindestens 0,5 % flüssiges Paraffin,

      wobei die Prozentangaben für das Gewicht des Gesamtgewichts der Zusammensetzung angegeben sind, wobei die Zusammensetzung weder Siliciumdioxid- noch Benzylalkoholpartikel umfasst.

2. Zusammensetzung zur Verwendung nach Anspruch 1, **dadurch gekennzeichnet, dass** sie zu mindestens 80 % Isopropylmyristat, vorzugsweise zu zwischen 83 % und 92 % Isopropylmyristat, umfasst.

3. Zusammensetzung zur Verwendung nach einem der Ansprüche 1 oder 2, **dadurch gekennzeichnet, dass** das in der Zusammensetzung vorhandene Tensid zwischen 7,5 % und 15 Gew.-% der Zusammensetzung ausmacht.

**4.** Zusammensetzung zur Verwendung nach einem der Ansprüche 1 bis 3, **dadurch gekennzeichnet, dass** das Tensid Propylenglycolmonolaurat, das ein hydrophiles/lipophiles Gleichgewicht gleich 5 besitzt, ist.

**5.** Zusammensetzung zur Verwendung nach einem der vorstehenden Ansprüche, **dadurch gekennzeichnet, dass** die Zusammensetzung eine Kombination von mindestens zwei Tensiden umfasst, deren hydrophiles/lipophiles Gesamtgleichgewicht der Kombination kleiner als oder gleich 5 ist.

**6.** Zusammensetzung zur Verwendung nach einem der vorstehenden Ansprüche, **dadurch gekennzeichnet, dass** das flüssige Paraffin mindestens 2 Gew.-% der Zusammensetzung, vorzugsweise zwischen 2 % und 4 Gew.-% der Zusammensetzung, ausmacht.

**7.** Zusammensetzung zur Verwendung nach einem der vorstehenden Ansprüche, **dadurch gekennzeichnet, dass** das Tensid oder die Kombination von Tensiden aus kationischen, anionischen, amphoteren und nichtionischen Tensiden ausgewählt ist.

**8.** Zusammensetzung zur Verwendung nach einem der vorstehenden Ansprüche, **dadurch gekennzeichnet, dass** sie ferner mindestens ein Antioxidans und/oder mindestens eine pedikulizide Substanz und/oder mindestens eine Läuse abwehrende Substanz und/oder einen Gelbildner und/oder ein Konservierungsmittel umfasst.

**9.** Zusammensetzung zur Verwendung nach Anspruch 8, **dadurch gekennzeichnet, dass** das Antioxidans weniger als 1 Gew.-% der Zusammensetzung ausmacht und/oder aus Butylhydroxytoluol, Butylhydroxyanisol und Ascorbylpalmitat ausgewählt ist.

**10.** Zusammensetzung zur Verwendung nach Anspruch 8, **dadurch gekennzeichnet, dass** die pedikulizide Substanz weniger als 2 Gew.-% der Zusammensetzung ausmacht und/oder aus Malathion, Permethrinen, Pflanzenölen oder pedikuliziden natürlichen Ölen wie Anisöl, Teebaumöl, Myrtenöl, Lavendelöl, Neemöl und Hemmstoffen für das Wachstum von Nissen, wie Farnesol und Nerolidol, ausgewählt ist.

**11.** Zusammensetzung zur Verwendung nach Anspruch 8, **dadurch gekennzeichnet, dass** die abwehrende Substanz weniger als 5 Gew.-% der Zusammensetzung ausmacht und/oder aus Myrtenöl, Zitronengras, Ethylbutylacetylaminopropionat (IR 3535), DET (Diethyltoluamid), ätherischen Öle aus Zitrone, Lavendel, Rosengeranie, Cineol-Rosmarin, Sellerie und Teebaum ausgewählt ist.

**12.** Zusammensetzung zur Verwendung nach einem der vorstehenden Ansprüche, **dadurch gekennzeichnet, dass** sie in Form einer Lotion oder eines Sprays verwendet wird.

**13.** Zusammensetzung zur Verwendung nach Anspruch 8, **dadurch gekennzeichnet, dass** das Geliermittel 5 Gew.-% der Zusammensetzung ausmacht und/oder aus Copolymeren von Siloxan und Polyamiden ausgewählt ist.

**14.** Zusammensetzung zur Verwendung nach Anspruch 8, **dadurch gekennzeichnet, dass** das Konservierungsmittel weniger als 3 Gew.-% der Zusammensetzung ausmacht.

**15.** Zusammensetzung zur Verwendung nach einem der vorstehenden Ansprüche, **dadurch gekennzeichnet, dass** sie für eine topische Anwendung auf der Kopfhaut und dem Haar bei der Vorbeugung oder der Behandlung von Pedikulose des Kopfs beim Menschen oder bei tierischem Fell bei der Vorbeugung oder der Behandlung von tierischer Pedikulose verwendet wird.

**Claims**

**1.** Composition in the form of a rinsable oily solution for use in the prevention or treatment of pediculosis, comprising:

   - at least 70% isopropyl myristate,
   - at least 7.5% of at least one surfactant with a hydrophilic-lipophilic balance of less than or equal to 5, and
   - at least 0.5% liquid paraffin,

the percentages being given by weight of the total weight of the composition,
said composition comprising neither silica particles nor benzyl alcohol.

2. The composition for use according to claim 1, **characterized in that** it comprises at least 80% isopropyl myristate, preferentially between 83% and 92% isopropyl myristate.

3. The composition for use according to one of claims 1 or 2, **characterized in that** the surfactant present in the composition represents between 7.5% and 15% by weight of said composition.

4. The composition for use according to one of claims 1 to 3, **characterized in that** the surfactant is propylene glycol monolaurate having a hydrophilic-lipophilic balance equal to 5.

5. The composition for use according to any one of the preceding claims, **characterized in that** the composition comprises a combination of at least two surfactants whose overall hydrophilic-lipophilic balance of said combination is less than or equal to 5.

6. The composition for use according to any one of the preceding claims, **characterized in that** the liquid paraffin represents at least 2% by weight of said composition, preferentially between 2% and 4% by weight of said composition.

7. The composition for use according to any one of the preceding claims, **characterized in that** the surfactant or the combination of surfactants is selected from cationic, anionic, amphoteric and nonionic surfactants.

8. The composition for use according to any one of the preceding claims, **characterized in that** it also comprises at least one antioxidant agent, and/or at least one pediculicidal substance, and/or at least one lice-repellent substance, and/or a gelling agent, and/or a preservative.

9. The composition for use according to claim 8, **characterized in that** the antioxidant agent represents less than 1% by weight of the composition and/or is selected from butyl hydroxytoluene, butyl hydroxyanisole and ascorbyl palmitate.

10. The composition for use according to claim 8, **characterized in that** the pediculicidal substance represents less than 2% by weight of the composition and/or is selected from malathion, permethrin, plant essences or natural pediculicidal oils such as anise oil, tea tree oil, myrtle oil, lavender oil, neem oil and nit growth inhibitors such as Farnesol and Nerolidol.

11. The composition for use according to claim 8, **characterized in that** the repellent substance represents less than 5% by weight of the composition and/or is selected from myrtle oil, citronella, ethyl butylacetylaminopropionate (IR3535), DEET (Diethyltoluamide), lemon, lavender, rose geranium, rosemary cineole, celery and tea-tree essential oils.

12. The composition for use according to any one of the preceding claims, **characterized in that** it is used in the form of a lotion or spray.

13. The composition for use according to claim 8, **characterized in that** the gelling agent represents 5% by weight of the composition and/or is selected from copolymers of siloxane and polyamides.

14. The composition for use according to claim 8, **characterized in that** the preservative represents less than 3% by weight of the composition.

15. The composition for use according to any one of the preceding claims, **characterized in that** it is used for topical application to the scalp and hair in the prevention or treatment of pediculosis of the head in humans or to the animal's coat in the prevention or treatment of animal pediculosis.

**EP 4 058 009 B1**

**RÉFÉRENCES CITÉES DANS LA DESCRIPTION**

**Documents brevets cités dans la description**

- WO 0119190 A
- EP 1499184 A
- FR 0955753
- WO 2019053134 A

**Littérature non-brevet citée dans la description**

- *CHEMICAL ABSTRACTS,* 110-27-0